# NOUVEAU FASCICULE DE BREVET EUROPEEN

(11) **EP 0 966 248 B2**
(45) Date de publication et mention de la décision concernant l'opposition: **02.04.2008**
(45) Mention de la délivrance du brevet: 12.11.2003
(21) Numéro de dépôt: 98959951.9
(22) Date de dépôt: 10.12.1998
(51) Int. Cl.: A61K 8/81, A61K 8/89, A61Q 5/12

(54) **COMPOSITIONS POUR LE TRAITEMENT DES MATIERES KERATINIQUES CONTENANT L'ASSOCIATION D'UN POLYMERE ZWITTERIONIQUE ET D'UNE SILICONE NON-VOLATILE ET INSOLUBLE DANS L'EAU**
ZUSAMMENSETZUNGEN ZUR BEHANDLUNG VON KERATINMATERIAL ENTHALTEND EIN GEMISCH EINES ZWITTERIONISCHEN POLYMERS UND EINES NICHTFLÜCHTIGEN UND WASSERUNLÖSLICHEN SILIKONÖLS
COMPOSITIONS FOR TREATING KERATINOUS MATERIALS CONTAINING A COMBINATION OF A ZWITTERION POLYMER AND A WATER INSOLUBLE NON-VOLATILE SILICON

(30) Priorité: 31.12.1997 FR 9716807
(43) Date de publication de la demande: 29.12.1999
(73) Titulaire: L'ORÉAL, 75008 Paris (FR)
(72) Inventeur: RESTLE, Serge, F-95390 Saint Prix (FR); CAUWET-MARTIN, Danièle, F-75011 Paris (FR)
(74) Mandataire: Le Blainvaux Bellegarde, Françoise
(86) Numéro de dépôt international: PCT/FR1998/002688
(87) Numéro de publication internationale: WO 1999/034770

(56) Documents cités:
- EP-A- 0 014 479
- EP-A- 0 535 447
- EP-A- 0 604 717
- EP-A- 0 761 206
- WO-A-91/03229
- WO-A-97/35544
- WO-A-99/22698
- DE-A- 4 401 708
- Datenblätter der Dow Corning zu Dow Corning 200 und Dow Corning 1401

## Description

La présente invention est relative à de nouvelles compositions pour le traitement des matières kératiniques, en particulier des cheveux contenant l'association d'un polymère zwittérionique et d'une silicone non aminée, non-volatile, non-hydrosoluble ainsi que leurs utilisations et procédés.

On recherche depuis quelques années dans le domaine des shampooings et après-shampooings des produits conditionneurs des cheveux permettant d'apporter des effets cosmétiques supplémentaires après application et rinçage tels que la douceur, la souplesse, un bon démêlage, un effet brillant et/ou un effet coiffant.

On connaît dans l'art antérieur que les silicones sont des produits cosmétiques particulièrement recherchés dans les formulations capillaires pour leurs propriétés de conditionnement des cheveux, leurs propriétés adoucissantes, démêlantes et leur apport de brillance. Ces ingrédients présentent l'inconvénient, lorsqu'ils sont utilisés dans des compositions capillaires rincées de se déposer difficilement et de façon irrégulière sur les cheveux à l'application et après rinçage et de ne pas pouvoir apporter aux cheveux les effets recherchés propres aux silicones.

On a envisagé dans la demande de brevet WO91/03229 des compositions shampooings à base de polymères zwittérioniques comprenant un monomère de type ammonium quaternaire et un monomère de type acide carboxylique. Ces polymères utilisés seuls ne permettent pas encore d'obtenir des propriétés cosmétiques satisfaisantes.

On a également envisagé dans le brevet US5650383 d'associer des silicones à un polymère amphotère du type acide acrylique/chlorure de diallyldiméthyl ammonium. Ces associations ne donnent pas encore entière satisfaction.
La demande EP761206 décrit des compositions détergentes comprenant une base lavante, un alcool gras, une silicone aminée et un poymère cationique.
La demande WO97/35544 décrit des shampooings comprenant un tensioactif anionique et un polymère cationique réticulé.
La demande WO99/22698 opposable au titre de la nouveauté selon l'article 54(3) CBE décrit des compositions non lavantes comprenant une microémulsion de silicone et du polyquaternium-47.

Le document DE 44 01 708 decrit des concentrats de polymere de type cationique, zwitterionique, amphotère ou non ionique pour la preparation de compositions cosmetiques.

La demanderesse a maintenant découvert qu'en associant des polymères zwittérioniques particuliers à des silicones non aminées non volatiles, non solubles dans l'eau, de viscosité que l'on définira plus loin, on pouvait obtenir de manière surprenante des compositions cosmétiques à base de silicones non aminées ne présentant pas les inconvénients évoqués ci-dessus, produisant de meilleures performances cosmétiques notamment au niveau du toucher et du démêlage ainsi que de bonnes propriétés coiffantes.

En particulier, cette association favorise le dépôt de silicone sur les matières kératiniques.

D'autre part, la demanderesse a découvert de manière inattendue qu'en associant les silicones de l'invention aux polymères zwittérioniques définis ci-dessous, on améliorait nettement les performances cosmétiques du polymère zwittérionique.

L'invention a donc pour objet des compositions cosmétiques ou dermatologiques caractérisées par le fait qu'elles comprennent dans un milieu aqueux cosmétiquement et/ou dermatologiquement acceptable au moins :
a) un polymère zwittérionique résultant de la copolymérisation d'au moins un monomère de formule (I): 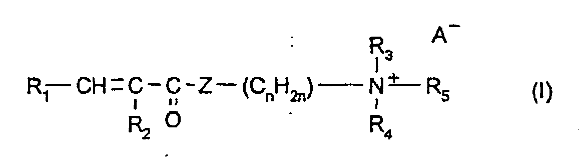 dans laquelle R₁ et R₂, identiques ou différents, représentent un atome d'hydrogène ou un radical méthyle, R₃, R₄ et R₅, identiques ou différents, représente un radical alkyle linéaire ou ramifié ayant de 1 à 4 atomes de carbone,
   Z représente un groupe NH ou un atome d'oxygène,
   n est un nombre entier de 2 à 5,
   A⁻ est un anion issu d'un acide organique ou minéral,
   et d'au moins un monomère de formule (II) :

   R₆―CH=CR₇―COOH (II)

   dans laquelle R₆ et R₇, identiques ou différents, représentent un atome d'hydrogène ou un radical méthyle;
   ledit polymère étant soluble dans l'eau à 1% en poids à pH inférieur à 6 et à 20°C;
b) une silicone non aminée, non-volatile, non-hydrosoluble de viscosité supérieure à 300 centistokes choisie parmi les polyalkylsiloxanes,
   à l'exclusion des compositions citées selon la revendication 1.

L'invention a également pour objet l'utilisation d'un polymère zwittérionique défini ci-dessus dans une composition comprenant une silicone polyalkylsiloxane de viscosité supérieure à 300 centistokes non aminée, non-volatile et insoluble dans l'eau pour augmenter l'effet conditionneur de la silicone.

L'invention a également pour objet une composition cosmétique ou dermatologique, caractérisée par le fait qu'elle comprend dans un milieu aqueux cosmétiquement et/ou dermatologiquement acceptable au moins :
a) un polymère zwittérionique résultant de la copolymérisation d'au moins un monomère de formule (I) : 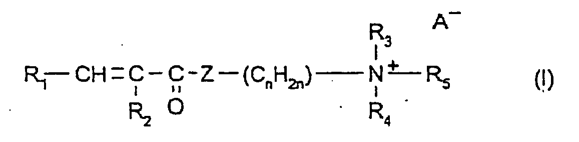 dans laquelle R₁ et R₂, identiques ou différents, représentent un atome d'hydrogène ou un radical méthyle, R₃, R₄ et R₅, identiques ou différents, représente un radical alkyle linéaire ou ramifié ayant de 1 à 4 atomes de carbone,
   Z représente un groupe NH ou un atome d'oxygène,
   n est un nombre entier de 2 à 5,
   A⁻ est un anion issu d'un acide organique ou minéral,
   et d'au moins un monomère de formule (II) :

   R₆―CH=CR₇―COOH (II)

   dans laquelle R₆ et R₇, identiques ou différents, représentent un atome d'hydrogène ou un radical méthyle;
   ledit polymère étant soluble dans l'eau à 1% en poids à pH inférieur à 6 et à 20°C;
b) une silicone polyalkylsiloxane non aminée, non-volatile, non-hydrosoluble de viscosité supérieure à 300 centistokes,
c) 4 à 30% en poids du poids total de la composition d'une base lavante constituée d'au moins un tensioactif ou d'un mélange de tensioactifs choisis dans le groupe des tensioactifs anioniques, cationiques, non-ioniques, amphotères ou zwittérioniques.

Selon la présente invention, par matières kératiniques, on comprend les cheveux, les cils, les sourcils, la peau, les ongles, les muqueuses ou le cuir chevelu et plus particulièrement les cheveux.

Les monomères de formule (I) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, quaternisés par exemple par un halogénure d'alkyle en C₁-C₄ ou un sulfate de dialkyle en C₁-C₄.

Plus particulièrement, le monomère de formule (I) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique.
Plus particulièrement, le monomère de formule (II) est l'acide acrylique.

Les monomères constituant les polymères zwittérioniques de l'invention sont de préférence déjà neutralisés et/ou quaternisés.

Le rapport du nombre de charges cationique/charge anionique est généralement inférieur à 1.

Les poids moléculaires moyen en poids des polymères zwittérioniques peuvent varier de 500 à 50.000.000 et sont de préférence compris entre 10.000 et 5 000 000.

Les polymères selon l'invention peuvent également contenir d'autre monomères tels que des monomères non ioniques et en particulier tels que les acrylates ou méthacrylates d'alkyle en C₁-C₄.

Les polymères zwittérioniques selon l'invention sont par exemple décrits dans la demande de brevet DE 39 29 973.

Les polymères zwittérioniques particulièrement préférés selon l'invention sont choisis parmi les copolymères acide acrylique/chlorure d'acrylamidopropyl triméthyl ammonium et les copolymères acide acrylique/chlorure de méthacrylamidopropyl triméthyl ammonium.

On peut également utiliser les terpolymères acide acrylique/chlorure d'acrylamidopropyl triméthyl ammonium / acrylate de méthyle par exemple commercialisés sous la dénomination MERQUAT 2001 par la société CALGON.

Les silicones de l'invention présentent de préférence une viscosité supérieure à 500 centistokes et plus particulièrement supérieure à 1000 centistokes. De préférence, les silicones ont une viscosité inférieure à 50.000.000 mm²/s. Les viscosités sont mesurées à 25°C.

Selon l'invention, on désigne par silicone non aminée toute silicone ne comportant pas au moins une aminé primaire ou un groupement ammonium quaternaire.

Parmi les polyalkylsiloxanes, on peut citer principalement :
- les polydiméthylsiloxanes linéaires à groupements terminaux triméthylsilyle, comme par exemple, et à titre non limitatif, les huiles SILBIONE de la série 70047 commercialisées par RHONE POULENC, l'huile 47 V 500 000 de RHONE POULENC ou certaines VISCASIL de la GENERAL ELECTRIC ;
- les polydiméthylsiloxanes linéaires à groupements terminaux hydroxydiméthylsilyle telles que les huiles de la série 48 V de RHONE POULENC.

Dans cette classe de polyalkylsiloxanes, on peut également mentionner les polyalkylsiloxanes vendus par la société GOLDSCHMIDT sous les dénominations commerciales ABILWAX 9800 et ABILWAX 9801 qui sont des polyalkyl(C₁-C₂₀)siloxanes.

Les polymères zwittérioniques de l'invention sont présents dans les compositions de l'invention dans des proportions allant de préférence de 0,01 à 20 % en poids et plus particulièrement de 0,1 à 10 % en poids par rapport au poids total de la composition.

Les silicones de l'invention sont généralement présentes dans les compositions dans des proportions allant de 0,001 à 20 % en poids et de préférence de 0,01 à 10 % en poids par rapport au poids total de la composition.

Le pH des compositions aqueuses conformes à l'invention est ajusté de préférence entre 3 et 11 et plus particulièrement entre 5 et 9 par l'emploi d'agents alcalinisants ou acidifiants ou de tampons.

Les compositions selon l'invention, lorsqu'elles se présentent en particulier sous forme de shampooing comprennent une base lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques.

La quantité minimale de base lavante est celle juste suffisante pour conférer à la composition finale un pouvoir moussant et/ou détergent satisfaisant.

Ainsi, selon l'invention, la base lavante peut représenter de 4 % à 30 % en poids, de préférence de 10 % à 25 % en poids, et encore plus préférentiellement de 12 % à 20 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.
Parmi les tensioactifs anioniques, on préfère utiliser selon l'invention les sels d'alkylsulfates et d'alkyléthersufates et leurs mélanges.

### (ii) Tensioactifs) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les aminés grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s):

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'aminés, on peut citer les produits vendus sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂ -CONHCH₂CH₂ -N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C₂M concentré par la Société MIRANOL.

### (iv) Tensioactifs cationiques :

Parmi les tensioactifs cationiques, dont la nature ne revet pas dans le cadre de la présente invention de caractère critique, on peut citer en particulier (liste non limitative) : les sels d'amines grasses primaires, secondaires ou tertiaires, éventuellement polyoxy-alkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Le milieu cosmétiquement ou dermatologiquement acceptable des compositions de l'invention est de préférence de l'eau ou une solution hydroalcoolique d'un alcool inférieur tels que l'éthanol, l'isopropanol ou le butanol.

La composition de l'invention peut également contenir au moins un additif choisi parmi les séquestrants, les adoucissants, les modificateurs de mousse, les colorants, les agents nacrants, les agents hydratants, les agents antipelliculaires ou anti-séborrhéiques, les agents de mise en suspension, les céramides, les pseudocéramides, les acides gras à chaînes linéaires ou ramifiées en C₁₆-C₄₀, les hydroxyacides, les électrolytes, les épaississants, les esters d'acides gras, les esters d'acides gras et de glycérol, les tensioactifs, les parfums, les conservateurs, les filtres solaires siliconés ou non siliconés, les protéines, les vitamines, les polymères ioniques ou non ioniques, les silicones hydrosolubles, les huiles végétales, animales, minérales ou synthétiques et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent se présenter sous la forme de liquides plus ou moins épaissis, de crèmes ou de gels et elles conviennent principalement au lavage, au soin et/ou au coiffage des cheveux. Elles peuvent aussi se présenter sous la forme de lotions à rincer.

Un autre objet de l'invention consiste en un procédé de traitement cosmétique des matières kératiniques telles que les cheveux caractérisé par le fait qu'on applique directement sur lesdites matières mouillés une composition telle que définie ci-dessus, et après un éventuel temps de pause, qu'on effectue un rinçage à l'eau ; ledit procédé pouvant être répété plusieurs fois.

Comme indiqué précédemment, les compositions selon l'invention confèrent aux cheveux, après rinçage, un remarquable effet conditionneur qui se manifeste notamment par une facilité de démêlage des cheveux, et des propriétés de douceur, de lissage des cheveux , une légèreté et du volume.

Les exemples qui suivent servent à illustrer la présente invention sans toutefois présenter un caractère limitatif.

### EXEMPLE 1

On a réalisé deux compositions de shampooing, l'une conforme à l'invention (composition A) et l'autre comparative (composition B):

| | A | B |
|---|---|---|
| | Invention | Comparatif |
| - Lauryléthersulfate de sodium (C₁₂/C₁₄ à 70/30) à 2,2 moles d'oxyde d'éthylène | 3 gMA | 3 gMA |
| Lauryl amido ether(3 OE) carboxylate de sodium à 30% de MA (AKYPO FOAM 30 BV de KAO) | 12 gMA | 12 gMA |
| - Silicone (60 000 cSt) (*) | 2 g | 2 g |
| - Copolymère de chlorure de diallyl diméthylammonium et d'acide acrylique (MERQUAT 280 de CALGON) | ― | 2 gMA |
| - Copolymère acide acrylique / chlorure de méthacrylamidopropyl triméthylammonium en solution aqueuse à 10% de MA | 2 gMA | ― |
| Mélange 1-(hexa décyloxy)-2-octadécanol/alcool cétylique | 2,5 g | 2,5 g |
| - Monoisopropanolamide d'acides de coprah | 1,5 g | 1,5 g |
| - NaOH qs pH | | 7,5 |
| - HCl qs pH | 7,5 | |
| - Eau déminéralisée qsp | 100 g | 100 g |

| | | |
|---|---|---|
| (*) : Polydiméthylsiloxane de viscosité 60 000 cSt commercialisé par la société DOW CORNING sous la dénomination FLUID DC 200 - 60 000 cSt. | | |

On effectue un shampooing en appliquant environ 1 g de la composition A sur des mèches de 2,5 g de cheveux naturels préalablement mouillés. On fait mousser le shampooing, on laisse pauser pendant 10 minutes puis on rince abondamment à l'eau. On sèche les mèches à 60°C pendant 30 minutes.
On procède selon le même mode opératoire que ci-dessus avec la composition comparative B.

Analytiquement, on a montré que le dépôt de la silicone sur les cheveux est supérieure avec la composition A selon l'invention.

### Exemple 2 :

On prépare un shampooing de composition suivante :

| | |
|---|---|
| Alkyl C₉/C₁₁ polyglugoside (1.4) en solution aqueuse à 40% vendu par KAO sous le nom de KAG 40 | 10g MA |
| Lauryl amido ether(3 OE) carboxylate de sodium à 30% de MA dans l'eau vendu par KAO sous le nom d'AKYPO FOAM 30 BV | 5g MA |
| Terpolymère acide acrylique/chlorure de méthacrylamidopropyl triméthyl-ammonium/acrylate de méthyle en solution aqueuse à 20% vendu sous la dénomination de MERQUAT 2001 par CALGON | 1g MA |
| Polydiméthylsiloxane (viscosité 250000 Cst) vendu sous la dénomination de MIRASIL DM 500000 par la société RHONE POULENC | 1g |
| Monoisopropanolamide d'acides de coprah | 2g |
| Mélange 1-(hexa décyloxy)-2-octadécanol/alcool cétylique | 2.5g |
| Acide polyacrylique reticulé vendu sous le nom de CARBOPOL 980 par GOODRICH | 0,5 g |
| Conservateurs, parfum qs | |
| Eau q.s.p. | 100g |
| pH ajusté à 6 (Na OH) | |

On obtient un shampooing présentant de bonnes propriétés moussantes, un effet coiffant et qui confère au cheveux secs un toucher lisse et doux.

## Revendications

1. Composition cosmétique ou dermatologique, **caractérisée par le fait qu'**elle comprend dans un milieu aqueux cosmétiquement et/ou dermatologiquement acceptable au moins :
a) un polymère zwittérionique résultant de la copolymérisation d'au moins un monomère de formule (I): 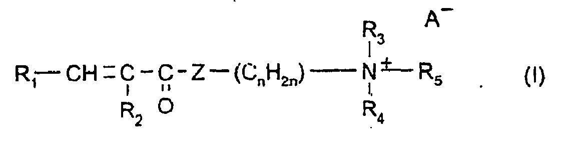 dans laquelle R₁ et R₂, identiques ou différents, représentent un atome d'hydrogène ou un radical méthyle, R₃, R₄ et R₅, identiques ou différents, représente un radical alkyle linéaire ou ramifié ayant de 1 à 4 atomes de carbone,
Z représente un groupe NH ou un atome d'oxygène,
n est un nombre entier de 2 à 5,
A⁻ est un anion issu d'un acide organique ou minéral,
et d'au moins un monomère de formule (II) :
R₆―C=-CR₇―COOH (II)
dans laquelle R₆ et R₇, identiques ou différents, représentent un atome d'hydrogène ou un radical méthyle;
ledit polymère étant soluble dans l'eau à 1% en poids à pH inférieur à 6 et à 20°C;
b) une silicone non aminée, non-volatile, non-hydrosoluble de viscosité supérieure à 300 centistokes choisie parmi les polyalkylsiloxanes,
à l'exclusion des 4 compositions ci-dessous:
L'exemple suivant est une composition gélifiante:
| Composants | Poids (%) |
|---|---|
| | |
| PVP¹ | 2.00 |
| copolymère PVP/VA (50% actif)² | 3.00 |
| Isoteareth 20³ | 0.30 |
| Parfum | 0.20 |
| Polyquaterium -47⁴ | 0.50 |
| Eeau | 88.60 |
| Propylène Glycol | 0.20 |
| Glycérine | 0.25 |
| Hydroxyethyl Cellulose⁵ | 1.00 |
| Microémulsion de silicone (25 %actif)⁶ | 3.00 |
| Conservateurs | 0.95 |
| | |
|---|---|
| 1 PVP K-30, ISP 2.Luviskol VA 73W, BASF 3. Arosurf 66 E-20, Witco 4. Merquat - 2001, Calgon 5.Cellosize PCG-10, Amerchol 6. DC1550 microémulsion siliconée de Dow Coming avec une taille de particule de 50 nm, un sytème tensio-actif anionique/nonionique et une silicone avec une phase interne de viscosité =100,000 cps. | |
L'exemple suivant est une composition moussante:
| Composition concentrée | |
|---|---|
| ingrédients | Poids (%) |
| Ethanol (200 proof) | 6.00 |
| Ester Butylique de copolymère PVM/MA (50% actif)¹ | 4.00 |
| PVP/VA (50% actif)² | 2.00 |
| Parfum | 0.10 |
| Eau | 83.19 |
| Aminométhyl propanol³ | 0.10 |
| Polyquaterium -47⁴ | 0.30 |
| Lauramide DEA⁵ | 0.30 |
| Steareth 21⁶ | 0.10 |
| Microémulsion siliconée (25% actif) | 3.00 |
| Conservateurs | 0.91 |
| | |
|---|---|
| 1. Gantrez ES-425 ISP 2. PVP, 735W, ISP 3. AMP, ordinaire, Angus 4. Merquat - 2001, Calgon 5. Monamide 716, Mona 6. BRIJ 721, ICI 7. DC2-5791 microémulsion siliconée de Dow Coming avec une taille de particule de 49 nm, un système tensioactif anionique/nonionique et une silicone avec une phase interne de viscosité = 80.000 cps | |
L'exemple suivant est une lotion anti bactérienne
| Ingrédients | Poids (%) |
|---|---|
| Eau | 75.69 |
| Ethanol | 20.00 |
| LaurylSulfate d'ammonium | 0.60 |
| Triclosan¹ | 0.15 |
| Propylène glycol | 0.20 |
| Mélange de Polyacrylate et de copolymère de polyacrylate ² | 1.00 |
| Parfum | 0.06 |
| Polyquaterium -47 (20% actif) ³ | 0.50 |
| Hydroxylpropyl Guar ⁴ | 0.50 |
| Microémulsion de silicone (25% actif)⁵ | 1.00 |
| Conservateurs | 0.30 |
| | |
|---|---|
| 1. Irgasan DP 300, CIBA- Geigy 2. Acusol 445, Rhom & Haas 3. Merquat - 2001, Calgon 4. Jaguar HP 105, Rhone-Poulenc 5. DC-1845 Microémulsion de silicone de Dow Coming avec une taille de particule de 33 nm, un système tensioactif anionique/nonionique et une silicone avec une phase interne de viscosité = 77.000 cps | |
L'exemple suivant est un shampooing transparent 2-in-1 comprenant :
| ingrédients | Poids (%) |
|---|---|
| Ethanol (200 proof) | 8.00 |
| Polyquaterium -47 (20% actif) ¹ | 5.00 |
| Diméthyl laurylamine ² | 1.20 |
| Isododécane ³ | 0.50 |
| Parfum | 0.10 |
| Eau | 64.04 |
| Laurylsulfate d'ammonium | 15.00 |
| Lauramide DEA ⁵ | 2.00 |
| Steareth 21 ⁶ | 0.250 |
| Microémulsion siliconée (25% actif) | 3.00 |
| Conservateurs | 0.91 |
| | |
|---|---|
| 1 Merquat - 2001, Calgon 2 C12 Alkyl Dimethylamine, AT-1295 LT 3 Permethyl 99A, Presperse Inc. 4 Monamide 716, Mona 5 BRIJ 721, ICI 6 DC2-5791 microémulsion de silicone de Dow Corning avec une taille de particule de 49 nm, un système tensioactif anionique/nonionique et une silicone avec une phase inteme de viscosité = 80.000 cps. | |

2. Composition selon la revendication 1, **caractérisée par le fait que** les silicones présentent une viscosité supérieure à 500 centistokes et plus particulièrement supérieure à 1000 centistokes.

3. Composition selon la revendication 1 ou 2, **caractérisée par le fait que** les polyalkylsiloxanes sont choisis dans le groupe constitué par :
- les polydiméthylsiloxanes linéaires à groupements terminaux triméthylsilyle,
- les polydiméthylsiloxanes linéaires à groupements terminaux hydroxydiméthyl silyle.

4. Composition selon l'une quelconque des revendications 1 à 3, **caractérisée par le fait que** la silicone est présente dans des proportions allant de 0,001 à 20 % en poids et de préférence de 0,01 à 10 % en poids par rapport au poids total de la composition.

5. Composition selon l'une quelconque des revendications 1 à 4, **caractérisée par le fait que** les monomères de formule (I) sont choisis dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, quaternisés par exemple par un halogénure d'alkyle en C₁-C₄ ou un sulfate de dialkyle en C₁-C₄.

6. Composition selon la revendication 5, **caractérisée en ce que** le monomère de formule (I) est choisi parmi le chlorure d'acrylamidopropyl triméthyl ammonium et le chlorure de méthacrylamidopropyl triméthyl ammonium.

7. Composition, selon l'une quelconque des revendications 1 à 6, **caractérisée en ce que** les monomères de formule (II) sont choisis dans le groupe constitué par l'acide acrylique, l'acide méthacrylique, l'acide crotonique et l'acide méthyl-1 crotonique.

8. Composition selon la revendication 7, **caractérisée en ce que** le monomère de formule (II) est l'acide acrylique.

9. Composition selon l'une quelconque des revendications 1 à 8, **caractérisée par le fait que** le polymère zwittérionique est choisi dans le groups constitué par les copolymères d'acide acrylique et de chlorure de d'acrylamidopropyl triméthyl ammonium et les copolymères acide acrylique/chlorure de méthacrylamidopropyl triméthl ammonium.

10. Composition selon l'une quelconque des revendications 1 à 9, **caractérisée par le fait que** le polymère zwittérionique est présent dans des concentrations allant de 0,01 à 20 % en poids et de préférence de 0,1 à 10 % en poids par rapport au poids total de la composition.

11. Composition selon l'une quelconque des revendications 1 à 10, **caractérisée par le fait que** le pH est ajusté entre 3 et 11.

12. Composition selon l'une quelconque des revendications 1 à 11, **caractérisée par le fait qu'**elle comporte en plus une base lavante constituée d'au moins un tensioactif ou d'un mélange de tensioactifs choisis dans le groupe des tensioactifs anioniques, cationiques, non-ioniques, amphotéres ou zwittérioniques.

13. Composition selon la revendication 12, **caractérisée par le fait que** la base lavante représente de 4 à 30 % en poids du poids total de la composition.

14. Composition selon l'une quelconque des revendications 1 à 13, **caractérisée par le fait que** le milieu cosmétiquement ou dermatologiquement acceptable est constituée d'eau ou d'un mélange d'eau et d'alcool inférieur choisi parmi l'éthanol, l'isopropanol ou le butanol.

15. Composition selon l'une quelconque des revendications 1 à 14, **caractérisée par le fait qu'**elle contient en au moins un additif choisi parmi les séquestrants, les adoucissants, les modificateurs de mousse, les colorants, les agents nacrants, les agents hydratants, les agents antipelliculaires ou anti-séborrhéiques, les agents de mise en suspension, les céramides, les pseudocéramides, les acides gras à chaînes linéaires ou ramifiées en C₁₈-C₄₀, les hydroxyacides, les électrolytes, les épaississants, les esters d'acides gras, les esters d'acides gras et de glycérol, les tensioactifs, les parfums, les conservateurs, les filtres solaires siliconés ou non siliconés, les protéines, les vitamines, les polymères ioniques ou non ioniques, les silicones hydrosolubles, les huiles végétales, animales, minérales ou synthétiques et tout autre additif classiquement utilisé dans le domaine cosmétique.

16. Composition selon l'une quelconque des revendications 1 à 15, **caractérisée par le fait qu'**elle est un produit à rincer pour le lavage, le soin et/ou le coiffage des cheveux.

17. Procédé de traitement cosmétique des matières kératiniques telles que les cheveux, **caractérisé par le fait qu'**on applique directement sur lesdites matières mouillées une composition selon l'une quelconque des revendications 1 à 16, que l'on effectue après un éventuel temps de pose, un rinçage à l'eau.

18. Utilisation d'un polymère zwittérionique défini selon la revendication 1 dans une composition comprenant une polyalkylsiloxane non aminée, non volatile et insoluble dans l'eau de viscosité supérieure à 300 centistokes pour augmenter l'effet conditionneur de la silicone.

19. Composition cosmétique ou dermatologique selon la revendication 1 **caractérisée par le fait qu'**elle comprend dans un milieu aqueux cosmétiquement et/ou dermatologiquement acceptable au moins:
a) un polymère zwittérionique résultant de la copolymérisation d'au moins un monomère de formule (I): 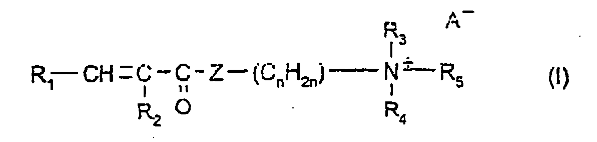 dans laquelle R₁ et R₂, identiques ou différents, représentent un atome d'hydrogène ou un radical méthyle, R₃, R₄ et R₅, identiques ou différents, représente un radical alkyle linéaire ou ramifié ayant de 1 à 4 atomes de carbone,
Z représente un groupe NH ou un atome d'oxygène,
n est un nombre entier de 2 à 5,
A- est un anion issu d'un acide organique ou minéral,
et d'au moins un monomère de formule (II) :
R₆―CH=CR₇―COOH (II)
dans laquelle R₆ et R₇, identiques où différents, représentent un atome d'hydrogène ou un radical méthyle;
ledit polymère étant soluble dans l'eau à 1% en poids à pH inférieur à 6 et à 20°C;
b) une silicone non aminée, non-volatile, non-hydrosoluble de viscosité supérieure à 300 centistokes, choisie parmi les polyalkylsiloxanes,
c) 4 à 30% en poids au poids total de la composition d'une base lavante constituée d'au moins un tensioactif ou d'un mélange de tensioactifs choisis dans le groupe des tensioactifs anioniques, cationiques, non-ioniques, amphotères ou zwittérioniques,
lesdits tensioactifs anioniques étant choisis parmi :
les sels des composés suivants : les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates , les alkylsulfoacétates ; les alkylétherphosphates; les aclsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle, les gels d'acides gras; les acyl-lactylates dont le radical acyle comporte 8 à 20 atomes de carbone, les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges..

## Claims

1. Cosmetic or dermatological composition, **characterized in that** it comprises, in a cosmetically and/or dermatologically acceptable aqueous medium, at least:
a) one zwitterionic polymer resulting from the copolymerization of at least one monomer of formula (I): 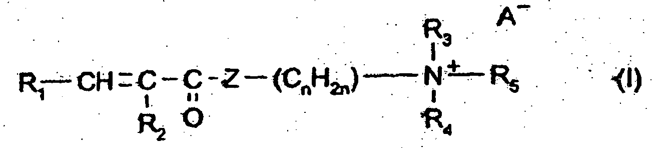 in which R₁ and R₂, which may be identical or different, represent a hydrogen atom or a methyl radical, and R₃, R₄ and R₅, which may be identical or different, represent a linear or branched alkyl radical containing from 1 to 4 carbon atoms,
Z represents an NH group or an oxygen atom,
n is an integer from 2 to 5,
A⁻ is an anion derived from an organic or inorganic acid,
and of at least one monomer of formula (II):
R₆-CH=CR₇-COOH **(II)**
in which R₆ and R₇, which may be identical or different, represent a hydrogen atom or a methyl radical;
the said polymer being soluble in water to a concentration of 1% by weight at a pH below 6 and at 20°C;
b) one non-amino, non-volatile, water-insoluble silicone with a viscosity of greater than 300 centistokes chosen from polyalkylsiloxanes with the exception of the 4 compositions below:
The following example is a gelling composition:
| Components | Weight (%) |
|---|---|
| | |
| PVP³ | 2.00 |
| PVP/VA copolymer (50% active agent)² | 3.00 |
| Isoteareth 20³ | 0.30 |
| Fragrance | 0.240 |
| Polyquaternium-47⁴ | 0.50 |
| Water | 88.60 |
| Propylene glycol | 0.20 |
| Glycerol | 0.25 |
| Hydroxyethylcellulose⁵ | 1.00 |
| Silicone microemulsion (25% active agent)⁶ | 3.00 |
| Preserving agents | ,0.95 |
| | |
|---|---|
| 1. PVP K-30, ISP 2. Luviskol VA 73W, BASF 3. Arosurf 66E-20, Witco 4. Merquat - 2001, Gallon 5. Cellosize PGG-10, Amerchol 6. DC1550 silicone microemulsion from Dow Corning, with a particle size of 50 nm, an anionic/nonionic surfactant system and a silicone with an inner phase of viscosity = 100,000 cps. | |
The following example is a foaming composition:
Concentrated composition
| Ingredients | Weight (%) |
|---|---|
| Ethanol (200 proof) | 6.00 |
| Butyl ester of PVM/MA copolymer (50% active agent)¹ | 4.00 |
| PVP/VA (50% active agent) ² | 2.00 |
| Fragrance | 0.10 |
| Water | 83.19 |
| Aminomethyl propanol 3 | 0.10 |
| Polyquaternium-47⁴ | 0.30 |
| Lauramide DEA⁵ | 0.30 |
| Steareth 21⁶ | 0.10 |
| Silicone microemulsion (25% active agent) | 3.00 |
| Preserving agents | 0.91 |
| | |
|---|---|
| 1. Gantrez ES-425 ISP 2. PVP, 735W, ISP 3. AMP, ordinary, Angus 4. Merquat - 2001, Calgon 5. Monamide 716, Mona 6 BRIJ 721, ICI 7. DC2-5791 silicone microemulsion from Dow Corning with a particle size of 49 nm, an anionic/nonionic surfactant system and a silicone with an inner phase of viscosity = 80,000 cps. | |
The following example is an antibacterial lotion:
| Ingredients | Weight (%) |
|---|---|
| Water | 75.69 |
| Ethanol | 20.00 |
| Ammonium lauryl sulphate | 0.60 |
| Triclosan¹ | 0.15 |
| Propylene glycol | 0.20 |
| Mixture of polyacrylate and of polyacrylate Copolymer ² | 1.00 |
| Fragrance | 0.06 |
| Polyquaternium-47 (20% active agent)³ | 0.50 |
| Hydroxypropylguar⁴ | 0.5.0 |
| Silicone microemulsion (25% active agent) ⁵ | 1.00 |
| Preserving agents | 0.30 |
| | |
|---|---|
| 1. Irgasan DP 300, CIBA-Geigy 2. Acusol 445, Rohm & Haas 3. Merquat - 2001, Calgon 4. Jaguar HP 105, Rhône-Poulenc 5. DC-1845 silicone microemulsion from Dow Corning, with a particle size of 33 nm, an anionic/nonionic surfactant system and a silicone with an inner phase of viscosity = 77,000 cps | |
The following example is a transparent 2-in-1 shampoo comprising:
| Ingredients | Weight (%) |
|---|---|
| Ethanol (200 proof) | 8.00 |
| Polyquaternium-47 (20% active agent)¹ | 5.00 |
| Dimethyllaurylamine ² | 1.20 |
| Isododecane³ | 0.50 |
| Fragrance | 0.10 |
| Water | 64.04 |
| Ammonium lauryl sulphate | 15.00 |
| Lauramide DEA⁵ | 2.00 |
| Steareth 21⁶ | 0.250 |
| Silicone microemulsion (25% active agent) | 3.00 |
| Preserving agents | 0.91 |
| | |
|---|---|
| 1. Merquat - 2001, Calgon 2. C12 Alkyldimethylamine, AT-1295 LT 3. Permethyl 99A, Presperse Inc. 4 . Monamide 716, Mona 5. BRIJ 721, ICI 6. DC2-5791 silicone microemulsion from Dow Corning, with a particle size of 49 nm, an anionic/nonionic surfactant system and a silicone with an inner phase of viscosity = 80,000 cps. | |

2. Composition according to Claim 1, **characterized in that** the silicones have a viscosity of greater than 5 x 10⁻⁵ m²/s (500 centistokes) and more particularly greater than 10 x 10⁻⁵ m² /s (1000 centistokes).

3. Composition according to Claim 1 or 2, **characterized in that** the polyalkylsiloxanes are chosen from the group consisting of:
- linear polydimethylsiloxanes containing trimethylsilyl end groups,
- linear polydimethylsiloxanes containing hydroxydimethylsilyl end groups.

4. Composition according to any one of Claims 1 to 3, **characterized in that** the silicone is present in proportions ranging from 0.001 to 20% by weight and preferably from 0.01 to 10% by weight relative to the total weight of the composition.

5. Composition according to any one of Claims 1 to 4, **characterized in that** the monomers of formula (I) are chosen from the group consisting of:
- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide, dimethylaminopropylacrylamide,
which are quaternized, for example, with a C₁-C₄ alkyl halide or a C₁-C₄ dialkyl sulphate.

6. Composition according to Claim 5, **characterized in that** the monomer of formula (I) is chosen from acrylamidopropyltrimethylammonium chloride and methacrylamidopropyltrimethylammonium chloride.

7. Composition according to any one of Claims 1 to 6, **characterized in that** the monomers of formula (II) are chosen from the group consisting of acrylic acid, methacrylic acid, crotonic acid and 2-methyl-crotonic acid.

8. Composition according to Claim 7, **characterized in that** the monomer of formula (II) is acrylic acid.

9. Composition according to any one of Claims 1 to 8, **characterized in that** the zwitterionic polymer is chosen from the group consisting of copolymers of acrylic acid and acrylamidopropyltrimethylammonium chloride and acrylic acid/methacrylamidopropyltrimethylarnmonium chloride copolymers.

10. Composition according to any one of Claims 1 to 9, **characterized in that** the zwitterionic polymer is present in concentrations ranging from 0.01 to 20% by weight and preferably from 0.1 to 10% by weight relative to the total weight of the composition.

11. Composition according to any one of Claims 1 to 10, **characterized in that** the pH is adjusted to between 3 and 11.

12. Composition according to any one of Claims 1 to 11, **characterized in that** it also comprises a washing base consisting of at least one surfactant or a mixture of surfactants chosen from the group of anionic, cationic, nonionic, amphoteric and zwitterionic surfactants.

13. Composition according to Claim 12, **characterized in that** the washing base represents from 4 to 30% by weight relative to the total weight of the composition.

14. Composition according to any one of Claims 1 to 13 ,**characterized in that** the cosmetically or dermatologically acceptable medium consists of water or of a mixture of water and lower alcohol chosen from ethanol, isopropanol or butanol.

15. Composition according to any one of Claims 1 to 14, **characterized in that** it contains at least one additive chosen from sequestering agents, softeners, foam modifiers, dyes, pearlescent agents, moisturizers, antidandruff agents, anti-seborrhoeic agents, suspending agents, ceramides, pseudoceramides; fatty acids containing linear or branched C₁₆-C₄₀ chains, hydroxy acids, electrolytes, thickeners, fatty acid esters, fatty acid esters of glycerol, surfactants, fragrances, preserving agents, silicone or non-silicone sunscreens, proteins, vitamins, ionic or nonionic polymers, water-soluble silicones, plant, animal, mineral or synthetic oils and any other additive conventionally used in the cosmetics field.

16. Composition according to any one of Claims 1 to 15, **characterized in that** it is a rinse-out product for washing, conditioning and/or styling the hair.

17. Cosmetic treatment process for keratin substances such as the hair, **characterized in that** a composition according to any one of Claims 1 to 16 is applied directly to the said wet substances and, after optionally leaving it thereon for a period of time, it is rinsed out with water.

18. Use of a zwitterionic polymer defined according to Claim 1, in a composition comprising a non-amino, non-volatile, water-insoluble polyalkylsiloxane with a viscosity of greater than 300 centistokes to enhance the conditioning effect of the silicone.

19. Cosmetic or dermatological composition according to Claim 1, **characterized in that** it comprises, in a cosmetically and/or dermatologically acceptable aqueous medium, at least:
a) one zwitterionic polymer resulting from the copolymerization of at least one monomer of formula (I): 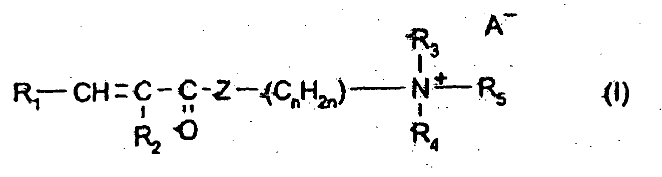 in which R₁ and R₂, which may be identical or different, represent a hydrogen atom or a methyl radical, R₃, R₄ and R₅, which may be identical or different, represent a linear or branched alkyl radical containing from 1 to 4 carbon atoms,
Z represents an NH group or an oxygen atom,
n is an integer from 2 to 5,
A⁻ is an anion derived from an organic or mineral acid,
and of at least one monomer of formula (II):
R₆-CH=CR₇-COOH (II)
in which R₆ and R₇, which may be identical or different, represent a hydrogen atom or a methyl radical;
the said polymer being soluble in water to a concentration of 1% by weight at a pH below 6 and at 20°C;
b) one non-amino, non-volatile, water-insoluble silicone with a viscosity of greater than 300 centistokes chosen from polyalkylsiloxanes
c) 4% to 30% by weight, relative to the total weight of the composition, of a washing base consisting of at least one surfactant or of a mixture of surfactants chosen from the group of anionic, cationic, nonionic, amphoteric or zwitterionic surfactants, the said anionic surfactants being chosen from:
the salts of the following compounds: alkyl ether sulphates, alkyl amido ether sulphates, alkyl aryl polyether sulphates, monoglyceride sulphates; alkyl sulphonates, alkyl phosphates, alkyl amido sulphonates, alkyl aryl sulphonates, α-olefin sulphonates and paraffin sulphonates; alkyl sulphosuccinates, alkyl ether sulphosuccinates, alkyl amide sulphosuccinates; alkyl sulphosuccinamates, alkyl sulfoacetates; alkyl ether phosphates; acyl sarcosinates; acyl isethionates and N-acyl taurates, the alkyl or acyl radical of all of these various compounds preferably containing from 8 to 24 carbon atoms, and the aryl radical preferably denoting a phenyl or benzyl group, fatty acid salts; acyl lactylates in which the acyl radical contains 8 to 20 carbon atoms, alkyl D galactoside uronic acids and salts thereof, polyoxyalkylenated carboxylic acid (C₆-C₂₄) alkyl ethers, polyoxyalkylenated carboxylic acid (C₆-C₂₄) alkylaryl ethers, polyoxyalkylenated carboxylic acid (C₆-C₂₄) alkylamido ethers and salts thereof, in particular those comprising from 2 to 50 ethylene oxide groups, and mixtures thereof.

## Patentansprüche

1. Kosmetische oder dermatologische Zusammensetzung, **dadurch gekennzeichnet, dass** sie in einem kosmetisch und/oder dermatologisch akzeptablen, wässrigen Medium zumindest enthält:
a) ein zwitterionisches Polymer, das durch Copolymerisation mindestens eines Monomers der folgenden Formel (I): 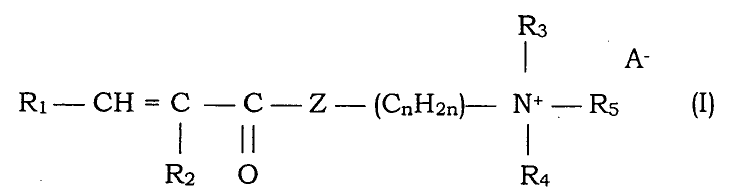 worin die Gruppen R₁ und R₂, die gleich oder verschieden sind, Wasserstoff oder Methyl bedeuten und die Gruppen R₃, R₄ und R₅, die gleich oder verschieden sind, eine geradkettige oder verzweigte Alkylgruppe mit 1 bis 4 Kohlenstoffatomen bedeuten,
Z eine NH-Gruppe oder ein Sauerstoffatom ist,
n eine ganze Zahl von 2 bis 5 bedeutet, und
A- ein Anion ist, das von einer anorganischen oder organischen Säure abgeleitet ist,
und mindestens eines Monomers der folgenden Formel (II) gebildet wird:
R₆ - CH = CR₇ - COOH (II),
worin die Gruppen R₆ und R₇, die gleich oder verschieden sind, Wasserstoff oder Methyl bedeuten;
wobei das Polymer bei einem pH-Wert unter 6 und einer Temperatur von 20 °C bei 1 Gew.-% in Wasser löslich ist; und
b) ein nicht aminiertes, nicht flüchtiges, nicht wasserlösliches Silicon mit einer Viskosität über 300 Centistokes ausgewählt unter Polyalkylsiloxanen,
wobei die vier folgenden Zusammensetzungen ausgenommen sind:
Das folgende Beispiel ist eine gelbildende Zusammensetzung:
| Komponenten | Gew.-% |
|---|---|
| | |
| PVP ¹ | 2,00 |
| PVP/VA-Coplymer (50 % Wirkstoff)² | 3,00 |
| Isosteareth 20³ | 0,30 |
| Parfum | 0,20 |
| Polyquaternium-47⁴ | 0,50 |
| Wasser | 88,60 |
| Propylenglykol | 0,20 |
| Glycerin | 0,25 |
| Hydroxyethylcelluloses | 1;00 |
| Silicon-Mikroemulsion (25 % Wirkstoff)⁶ | 3,00 |
| Konservierungsmittel | 0,95 |
| | |
|---|---|
| ¹ PVP K-30, ISP ² Luviskol VA 73W, BASF ³ Arosulf 66 E-20, Witco ⁴ Merquat-2001, Calgon ⁵ Cellosize PCG-10, Amerchol ⁶ DC 1550, siliconhaltige Mikroemulsion von Dow Corning mit einer Partikelgröße von 50 nm, anionisches/nichtionisches grenzflächenaktives System und Silicon mit einer inneren Phase mit einer Viskosität von 100 000 cP | |
Das folgende Beispiel ist eine schäumende Zusammensetzung:
Konzentrat
| Bestandteile | Gew.-% |
|---|---|
| | |
| Ethanol (200 proof) | 6,00 |
| PVM/MA-Copolymer, Butylester (50 % Wirkstoff)¹ | 4,00 |
| PVP/VA-Coplymer (50 % Wirkstoff)² | 2,00 |
| Parfum | 0,10 |
| Wasser | 83,19 |
| Aminomethylpropanol³ | 0,10 |
| Polyquaternium-47⁴ | 0,30 |
| Lauramid DEA⁵ | 0,30 |
| Steareth 21⁶ | 0,10 |
| Silicon-Mikroemulsion (25 % Wirkstoff)⁶ | 3,00 |
| Konservierungsmittel | 0,91 |
| | |
|---|---|
| ¹ Gantrez ES-425, ISP ² PVP 735W, ISP ³ übliches AMP, Angus ⁴ Merquat-2001, Calgon ⁵ Monamide 716, Mona ⁶ BRIJ 721, ICI ⁷ DC 2-5791, siliconhaltige Mikroemulsion von Dow Corning mit einer Partikelgröße von 49 nm, anionisches/nichtionisches grenzflächenaktives System und Silicon mit einer inneren Phase mit einer Viskosität von 80 000 cP | |
Das folgende Beispiel ist eine antibakterielle Lotion:
| Komponenten | Gew.-% |
|---|---|
| | |
| Wasser | 75,69 |
| Ethanol | 20,00 |
| Ammoniumlaurylsulfat | 0,60 |
| Triclosan¹ | 0,15 |
| Propylenglykol | 0,20 |
| Gemisch aus Polyacrylat und Poylacrylat-Copolymer² | 1,00 |
| Parfum | 0,60 |
| Polyquaternium-47 (20 % Wirkstoff)³ | 0,50 |
| Hydroxypropylguar⁴ | 0, 50 |
| Silicon-Mikroemulsion (25 % Wirkstoff)⁵ | 1,00 |
| Konservierungsmittel | 0,30 |
| | |
|---|---|
| ¹ Irgasan DP 300, Ciba Geigy ² Acusol 445, Rhom & Haas ³ Merquat-2001, Calgon ⁴ Jaguar HP 105, Rhone-Poulenc ⁵ DC-1845, siliconhaltige Mikroemulsion von Dow Corning mit einer Partikelgröße von 33 nm, anionisches/nichtionisches grenzflächenaktives System und Silicon mit einer inneren Phase mit einer Viskosität von 77 000 cP | |
Das folgende Beispiel ist ein transparentes 2-in-1-Shampoo:
Konzentrat
| Bestandteile | Gew.-% |
|---|---|
| | |
| Ethanol (200 proof) | 8,00 |
| Polyquaternium-47 (20 % Wirkstoff)¹ | 5,00 |
| Dimethyllaurylamin² | 1,20 |
| Isododecan³ | 0,50 |
| Parfum | 0,10 |
| Wasser | 64,04 |
| Ammoniumlaurylsulfat | 15,00 |
| Lauramid DEA⁵ | 2,00 |
| Steareth 216 | 0,250 |
| Silicon-Mikroemulsion (25 % Wirkstoff) | 3,00 |
| Konservierungsmittel | 0,91 |
| | |
|---|---|
| ¹ Merquat-2001, Calgon ² C12 Alkyldimethylamin, AT-1295 LT ³Permethyl 99A, Presperse Inc. ⁴ Monamide 716, Mona ⁵ BRIJ 721, ICI ⁶ DC 2-5791, siliconhaltige Mikroemulsion von Dow Corning mit einer Partikelgröße von 49 nm, anionisches/nichtionisches grenzflächenaktives System und Silicon mit einer inneren Phase mit einer Viskosität von 80 000 cP. | |

2. Zusammensetzung nach Anspruch 1, **dadurch gekennzeichnet, dass** die Silicone eine Viskosität über 5·10⁻⁵ m²/ s (500 Centistokes) und insbesondere über 10·10⁻⁵ m²/s (1000 Centistokes) besitzen.

3. Zusammensetzung nach Anspruch 1 oder 2, **dadurch gekennzeichnet, dass** die Polyalkylsiloxane ausgewählt sind unter:
- geradkettigen Polydimethylsiloxanen mit endständigen Trimethylsilylgruppen, und
- geradkettigen Polydimethylsiloxanen mit endständigen Hydroxydimethylsilylgruppen.

4. Zusammensetzung nach einem der Ansprüche 1 bis 3, **dadurch gekennzeichnet, dass** das Silicon in Mengenanteilen von 0,001 bis 20 Gew.-% und vorzugsweise 0,01 bis 10 Gew.-%, bezogen auf das Gesamtgewicht der Zusammensetzung, enthalten ist.

5. Zusammensetzung nach einem der Ansprüche 1 bis 4, **dadurch gekennzeichnet, dass** die Monomere der Formel (I) ausgewählt sind unter:
- Dimethylaminoethylmethacrylat, Dimethylaminoethylacrylat,
- Diethylaminoethylmethacrylat, Diethylaminoethylacrylat,
- Dimethylaminopropylmethacrylat, Dimethylaminopropylacrylat,
- Dimethylaminopropylmethacrylamid, Dimethylaminopropylacrylamid,
die beispielsweise mit einem C₁₋₄-Alkylhalogenid oder einem C₁₋₄-Dialkylsulfat quaternisiert sind.

6. Zusammensetzung nach Anspruch 5, **dadurch gekennzeichnet, dass** das Monomer der Formel (I) unter Acrylamidopropyltrimethylammoniumchlorid und Methacrylamidopropyltrimethylammoniumchlorid ausgewählt ist.

7. Zusammensetzung nach einem der Ansprüche 1 bis 6, **dadurch gekennzeichnet, dass** die Monomere der Formel (II) unter Acrylsäure, Methacrylsäure, Crotonsäure und 2-Methylcrotonsäure ausgewählt sind.

8. Zusammensetzung nach Anspruch 7, **dadurch gekennzeichnet, dass** das Monomer der Formel (II) die Acrylsäure ist.

9. Zusammensetzung nach einem der Ansprüche 1 bis 18, **dadurch gekennzeichnet, dass** das zwitterionische Polymer unter den Acrylsäure/Acrylamidopropyltrimethylammoniumchlorid-Copolymeren und den Acrylsäure/Methacrylamidopropyltrimethylammoniumchlorid-Copolymeren ausgewählt ist.

10. Zusammensetzung nach einem der Ansprüche 1 bis 9, **dadurch gekennzeichnet, dass** das zwitterionische Polymer in Konzentrationen von 0,01 bis 20 Gew.-% und vorzugsweise 0,1 bis 10 Gew.-%, bezogen auf das Gesamtgewicht der Zusammensetzung, enthalten ist.

11. Zusammensetzung nach einem der Ansprüche 1 bis 10, **dadurch gekennzeichnet, dass** der pH-Wert auf 3 bis 11 eingestellt ist.

12. Zusammensetzung nach einem der Ansprüche 1 bis 11, **dadurch gekennzeichnet, dass** sie sie ferner eine reinigende Basisformulierung enthält, die aus mindestens einem grenzflächenaktiven Stoff oder einem Gemisch von grenzflächenaktiven Stoffen besteht, die unter den anionischen, kationischen, nichtionischen, amphoteren oder zwitterionischen grenzflächenaktiven Stoffen ausgewählt sind.

13. Zusammensetzung nach Anspruch 12, **dadurch gekennzeichnet, dass** die reinigende Basisformulierung 4 bis 30 Gew.-% des Gesamtgewichts der Zusammensetzung ausmacht.

14. Zusammensetzung nach einem der Ansprüche 1 bis 13, **dadurch gekennzeichnet, dass** das kosmetisch oder dermatologisch akzeptable Medium aus Wasser oder einem Gemisch von Wasser und einem niederen Alkohol besteht, der unter Ethanol, Isopropanol oder Butanol ausgewählt ist.

15. Zusammensetzung nach einem der Ansprüche 1 bis 14, **dadurch gekennzeichnet, dass** sie mindestens einen Zusatzstoff enthält, der unter den Maskierungsmitteln, beruhigenden Stoffen, Schaummodifikatoren, Farbmitteln, Perlglanzpigmenten, Hydratisierungsmitteln, Wirkstoffen gegen Schuppen oder gegen Seborrhoe, Suspendiermitteln, Ceramiden, Pseudoceramiden, geradkettigen oder verzweigten Fettsäuren mit 16 bis 40 Kohlenstoffatomen, Hydroxysäuren, Elektrolyten, Verdickungsmitteln, Fettsäureestern, Fettsäure-GlycerinEstern, grenzflächenaktiven Stoffen, Parfums, Konservierungsmitteln, ggf. siliconhaltigen Sonnenschutzfiltern, Proteinen, Vitaminen, ionischen oder nichtionischen Polymeren, wasserlöslichen Siliconen, pflanzlichen, tierischen, mineralischen oder synthetischen Ölen oder beliebigen anderen, gewöhnlich in der Kosmetik verwendeten Zusatzstoffen ausgewählt ist.

16. Zusammensetzung nach einem der Ansprüche 1 bis 15, **dadurch gekennzeichnet, dass** es sich um ein Produkt, das ausgespült wird, für die Wäsche der Haare, für die Pflege der Haare und/oder für die Gestaltung der Haare handelt.

17. Verfahren zur kosmetischen Behandlung von Keratinsubstanzen, wie der Haare, das **dadurch gekennzeichnet ist, dass** auf die feuchten Keratinsubstanzen eine Zusammensetzung nach einem der Ansprüche 1 bis 16 direkt aufgetragen und anschließend gegebenenfalls nach einer Einwirkzeit mit Wasser gespült wird.

18. Verwendung eines zwitterionischen Polymers nach Anspruch 1 in einer Zusammensetzung, die ein nicht aminiertes, nicht flüchtiges, nicht wasserlösliches Polyalkylsiloxan mit einer Viskosität über 300 Centistokes enthält, um die Konditionierwirkung des Silicons zu verbessern.

19. Kosmetische oder dermatologische Zusammensetzung nach Anspruch 1, **dadurch gekennzeichnet, dass** sie in einem kosmetisch und/oder dermatologisch akzeptablen, wässrigen Medium enthält:
a) ein zwitterionisches Polymer, das durch Copolymerisation mindestens eines Monomers der folgenden Formel (I): 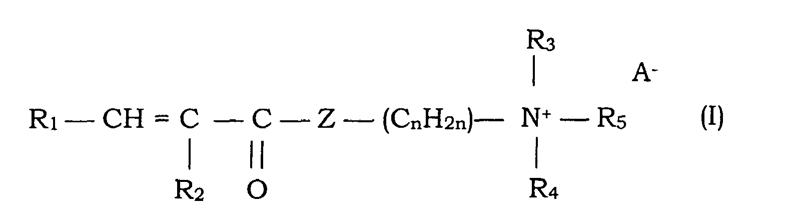 worin die Gruppen R₁ und R₂, die gleich oder verschieden sind, Wasserstoff oder Methyl bedeuten und die Gruppen R₃, R₄ und R₅, die gleich oder verschieden sind, eine geradkettige oder verzweigte Alkylgruppe mit 1 bis 4 Kohlenstoffatomen bedeuten,
Z eine NH-Gruppe oder ein Sauerstoffatom ist,
n eine ganze Zahl von 2 bis 5 bedeutet, und
A- ein Anion ist, das von einer anorganischen oder organischen Säure abgeleitet ist,
und mindestens eines Monomers der folgenden Formel (II) gebildet wird:
R₆ - CH = CR₇ - COOH (II),
worin die Gruppen R₆ und R₇, die gleich oder verschieden sind, Wasserstoff oder Methyl bedeuten;
wobei das Polymer bei einem pH-Wert unter 6 und einer Temperatur von 20 °C bei 1 Gew.-% in Wasser löslich ist; und
b) ein nicht aminiertes, nicht flüchtiges, nicht wasserlösliches Silicon mit einer Viskosität über 300 Centistokes, ausgewählt unter Polyalkylsiloxanen,
c) 4 bis 30 Gew.-% einer reinigenden Basisformulierung, bezogen auf das Gesamtgewicht der Zusammensetzung, die aus mindestens einem grenzflächenaktiven Stoff oder einem Gemisch von grenzflächenaktiven Stoffen besteht, die unter den anionischen, kationischen, nichtionischen, amphoteren oder zwitterionischen grenzflächenaktiven Stoffen ausgewählt sind,
wobei die anionischen grenzflächenaktiven Stoffe unter den Salzen der folgenden Verbindungen ausgewählt sind: Alkylethersulfaten, Alkylamidoethersulfaten, Alkylarylpolyethersulfaten, Monoglyceridsulfaten; Alkylsulfonaten, Alkylphosphaten, Alkylamidosulfonaten, Alkylarylsulfonaten, α-Olefinsulfonaten, Paraffinsulfonaten; Alkylsulfosuccinaten, Alkylethersulfosuccinaten, Alkylamidsulfosuccinaten; Alkylsulfosuccinamaten; Alkylsulfoacetaten; Alkyletherphosphaten; Acylsarcosinaten; Acylisethionaten und N-Acyltauraten, wobei die Alkyl- oder Acylgruppen der verschiedenen Verbindungen vorzugsweise 8 bis 24 Kohlenstoffatome enthalten und die Arylgruppe vorzugsweise Phenyl oder Benzyl bedeutet, Salzen von Fettsäuren; Acyllactylaten, deren Acylgruppe 8 bis 20 Kohlenstoffatome aufweist, Alkyl-D-galactosiduronsäuren und deren Salzen, polyalkoxylierten C₆₋₂₄-alkylcarbonsäureethern, polyalkoxylierten C₆₋₂₄-Alkylarylcarbonsäureethern, polyalkoxylierten C₆₋₂₄-Alkylamidocarbonsäureethern und ihren Salzen, insbesondere den Verbindungen, die 2 bis 50 Ethylenoxidgruppen aufweisen, und deren Gemischen.
